(19)

Europäisches
Patentamt
European
Patent Office
Office européen
des brevets

(11) **EP 4 356 754 A1**

(12) **EUROPEAN PATENT APPLICATION**
published in accordance with Art. 153(4) EPC

(43) Date of publication:
**24.04.2024 Bulletin 2024/17**

(21) Application number: **22804782.5**

(22) Date of filing: **20.05.2022**

(51) International Patent Classification (IPC):
**A23L 7/104** (2016.01)    **C12N 9/78** (2006.01)
**C12N 15/31** (2006.01)    **C12N 15/55** (2006.01)

(52) Cooperative Patent Classification (CPC):
**A23L 7/104; C07K 14/195; C12N 9/14; C12N 9/78**

(86) International application number:
**PCT/JP2022/020989**

(87) International publication number:
**WO 2022/244875 (24.11.2022 Gazette 2022/47)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **21.05.2021   JP 2021086113**

(71) Applicant: **Amano Enzyme Inc.
Nagoya-shi
Aichi 460-8630 (JP)**

(72) Inventor: **MATSUOKA, Tomohiro
Kakamigahara-shi, Gifu 509-0109 (JP)**

(74) Representative: **Müller-Boré & Partner
Patentanwälte PartG mbB
Friedenheimer Brücke 21
80639 München (DE)**

Remarks:
The complete document including Reference
Table(s) and the Sequence Listing(s) can be
downloaded from the EPO website

(54) **METHOD FOR MANUFACTURING PROCESSED OAT FOOD, BEVERAGE OR FOOD MATERIAL**

(57)    The purpose of the present invention is to provide a processing technique that enables protein deamidation while minimizing the degradation of oat-derived β-glucan during the processing of an oat food, beverage or food material. A method for manufacturing a processed oat food, beverage or food material, said method comprising a step for treating an unheated oat food, beverage or food material with a protein deamidase under temperature conditions of 65-75°C. This method enables protein deamidation while minimizing the degradation of oat-derived β-glucan.

**EP 4 356 754 A1**

**Description**

TECHNICAL FIELD

The present invention relates to a processing technique for an oat-food or drink product or food ingredient. Specifically, the present invention relates to a method for producing a processed oat-food or drink product or food ingredient, which enables a protein deamidation reaction while suppressing degradation of an oat-derived β-glucan.

BACKGROUND ART

Plant-based protein-containing food or drink products, which are rich in nutrients and have a long storage period, have been increasingly popular as alternatives to animal protein-containing food or drink products for various reasons such as the recent health boom, countermeasures against allergic problems, religious reasons, and increase in cases where people avoid outings accompanying the spread of infectious diseases.

Among them, oats are rich in nutrients such as protein, lipids, β-glucan, minerals, and the like, and thus have attracted attention for a long time as materials of plant-based protein-containing food or drink products, and processing methods thereof have been studied.

For example, Patent Document 1 discloses a technique of treating an oat suspension with β-amylase and α-amylase at about 54 to 57°C in order to obtain "enzyme preparations that hydrolyze cereal starch in a more cost-efficient and timely manner while producing a cereal suspension product that retains the flavoring and aromatic qualities of natural cereal and in which the viscosity, sugar content, and overall texture can be regulated or modified for a preferred end product".

On the other hand, since oats contain β-glucanase, the β-glucanase decreases β-glucan in the oat processing step. Thus, for the purpose of obtaining a β-glucan rich oat-food or drink product, it is necessary to deactivate β-glucanase.

Heat treatment is used for deactivation of β-glucanase. For example, Patent Document 2 discloses that oats are treated at 85 to 110°C in order to obtain a β-glucan rich composition.

PRIOR ART DOCUMENTS

PATENT DOCUMENTS

Patent Document 1: Japanese Patent Laid-open Publication No. 2002-527089
Patent Document 2: US Patent No. 7,494,683

SUMMARY OF THE INVENTION

PROBLEMS TO BE SOLVED BY THE INVENTION

In order to enable efficient intake of nutrients contained in oats, it is desired to provide processing that can, for example, increase the amount of soluble protein by solubilization of protein in addition to an increase in β-glucan by deactivation of β-glucanase.

Here, a protein deamidase may be used for processing food or drink product materials. The protein deamidase exhibits an action of degrading an amide group-containing side chain of a protein without cleavage of a peptide bond or crosslinking of the protein, and is used for solubilization of a protein in food or drink product materials by utilizing this action.

However, heat treatment used to deactivate β-glucanase for the purpose of increasing β-glucan will simultaneously deactivate the protein deamidase used for increasing soluble protein. That is, enzyme deactivation, which is a means for abundantly obtaining β-glucan is essentially incompatible with enzyme treatment, which is a means for obtaining an effect such as a solubilizing effect by a protein deamidation reaction.

It is therefore an object of the present invention to provide a processing technique that enables a protein deamidation reaction while suppressing degradation of an oat-derived β-glucan in processing of oat-food or drink products or food ingredients.

MEANS FOR SOLVING THE PROBLEM

The present inventor has unexpectedly found that β-glucanase can be deactivated without deactivating protein deamidase when the protein deamidase is allowed to act on an oat-food or drink product material under a very limited temperature condition of 65 to 75°C. The present invention has been completed by further conducting studies based on these findings.

That is, the present invention provides inventions of the following aspects.

Item 1. A method for producing a processed oat-food or drink product or food ingredient, the method including the step for treating an oat-food or drink product or food ingredient that has not been subjected to heat treatment, with a protein deamidase under a temperature condition of 65 to 75°C.

Item 2. The method according to item 1, in which the protein deamidase is a protein glutaminase derived from Chryseobacterium proteolyticum.

Item 3. The method according to item 1, in which the protein deamidase is a protein glutaminase including a polypeptide described in any of (1) to (3) below:

(1) a polypeptide having an amino acid sequence set forth in SEQ ID NO: 1 or 2,
(2) a polypeptide in which one or several amino acid residues are substituted, added, inserted, or deleted in the amino acid sequence set forth in SEQ ID NO: 1 or 2 and that exhibits heat resistance equivalent to heat resistance of a polypeptide having the amino acid sequence set forth in SEQ ID NO: 1 or 2; and
(3) a polypeptide having a sequence identity of 76% or more to the amino acid sequence set forth in SEQ ID NO: 1 or 2 and exhibiting heat resistance equivalent to heat resistance of a polypeptide having the amino acid sequence set forth in SEQ ID NO: 1 or 2.

Item 4. The method according to any one of items 1 to 3, in which the oat-food or drink product or food ingredient is oat milk.

Item 5. A protein deamidation agent that includes a protein deamidase and is used under a temperature condition of 65 to 75°C in order to perform protein deamidation of an oat-food or drink product or food ingredient that has not been subjected to heat treatment while suppressing degradation of β-glucan.

ADVANTAGES OF THE INVENTION

According to the present invention, there is provided a processing technique that enables a protein deamidation reaction while suppressing degradation of an oat-derived β-glucan in processing of oat-food or drink products or food ingredients.

BRIEF DESCRIPTION OF THE DRAWINGS

Fig. 1 is an SDS-PAGE image of a purified fraction of a protein glutaminase derived from strain 57594 of Chryseobacterium sp.
Fig. 2 is an SDS-PAGE image of a purified fraction of a protein glutaminase derived from strain 61798 of Chryseobacterium sp.
Fig. 3 shows results of a temperature stability test for protein glutaminases derived from strain 57594 and strain 61798 of Chryseobacterium sp.
Fig. 4 shows results of an optimal temperature test for protein glutaminases derived from strain 57594 and strain 61798 of Chryseobacterium sp.
Fig. 5 shows results of a pH stability test for protein glutaminases derived from strain 57594 and strain 61798 of Chryseobacterium sp.
Fig. 6 shows results of an optimal pH test for protein glutaminases derived from strain 57594 and strain 61798 of Chryseobacterium sp.
Fig. 7 shows the amount of β-glucan in the processed oat milk obtained in Test example.
Fig. 8 shows the protein deamidation rate of the processed oat milk obtained in Test example.

EMBODIMENTS OF THE INVENTION

1. Method for producing processed oat-food or drink product or food ingredient

The method for producing a processed oat-food or drink product or food ingredient of the present invention includes a step of treating an oat-food or drink product or food ingredient that has not been subjected to heat treatment, with a protein deamidase under a temperature condition of 65 to 75°C. Hereinafter, the method for producing a processed oat-food or drink product or food ingredient of the present invention will be described in detail.

## 1-1. Oat-food or drink product or food ingredient

In the present invention, the term "oat-food or drink product or food ingredient" refers to a material that is prepared for being subjected to the step of treating with a protein deamidase under a temperature condition of 65 to 75°C. In the present invention, an oat-food or drink product or food ingredient that has not been subjected to heat treatment in advance (hereinafter, also described as "pre-heat treatment") is used. The term "pre-heat treatment" used herein refers to a heat treatment performed for deactivating enzymes contained in the oat, which may be performed in the preparation of an oat-food or drink product or food ingredient. The term "enzyme" used herein encompasses at least β-glucanase, and may further encompass other endogenous enzymes such as lipase and lipoxygenase. The specific temperature for the pre-heat treatment is a temperature at which an enzyme as a deactivation target, such as β-glucanase, or lipase or lipoxygenase encompassed herein in addition to β-glucanase, can be deactivated. The temperature is determined according to the kind of the deactivation target, and thus can be appropriately selected by those skilled in the art. That is, as the oat-food or drink product or food ingredient that has not been subjected to the pre-heat treatment, one that has not had a thermal history by the pre-heat treatment at any stage until the oat is prepared into the form of the oat-food or drink product or food ingredient, is used.

The oat-food or drink product or food ingredient that has not been subjected to heat treatment (that is, pre-heat treatment) used in the present invention is specifically a beverage containing oat-derived protein and β-glucan (that is, one for drinking, oat beverage), a food containing oat-derived protein and β-glucan (that is, one for eating, oat food; in the present invention, the term "food" encompasses not only food for humans but also feed for animals other than humans), or a food ingredient containing oat-derived protein and β-glucan (that is, food ingredient that, in combination with other food materials, constitutes a beverage or a food; oat food ingredient).

The beverage containing oat-derived protein and β-glucan is not particularly limited as long as it is a liquid in which the oat-derived protein and β-glucan are dissolved and/or dispersed in water. Specific examples of the beverage containing oat-derived protein and β-glucan include (i) a liquid obtained by dispersing dry powder of a food material containing oat-derived protein and β-glucan in water; (ii) a liquid obtained by crushing and dispersing a food material containing oat-derived protein and β-glucan in water; (iii) a liquid obtained by, for example, removing at least a part of components other than the oat-derived protein from the liquid described in (i) or (ii) to increase the contents of the oat-derived protein and/or β-glucan; and (iv) a liquid obtained by dissolving and/or dispersing the dry powder prepared from any one of the liquids described in (i) to (iii) in water. Examples of the food material containing oat-derived protein and β-glucan include oat endosperm; oat endosperm with an aleurone layer; an oat aleurone layer and oat endosperm with hull; those with germ; and oat whole grains. As a preferred example of the beverage containing oat-derived protein and β-glucan, there may be mentioned oat milk. Examples of the oat milk include aqueous dispersions of oat flour.

As an example of the food containing oat-derived protein and β-glucan, there may be mentioned textured oat protein. The textured oat protein is a product obtained by texture-processing the food material containing oat-derived protein and β-glucan as a raw material with an extruder or the like so as to have a texture close to that of meat.

Examples of the food ingredient containing oat-derived protein and β-glucan include dry powders of the food materials containing oat-derived protein and β-glucan.

The protein content in the oat-food or drink product or food ingredient used in the present invention is not particularly limited, may vary depending on the properties of the food or drink product or food ingredient, and is, for example, 0.05 wt% or more, or 0.1 wt% or more, preferably 0.5 wt% or more, more preferably 1 wt% or more, still more preferably 1.25 wt% or more, or 1.5 wt% or more. The upper limit of the protein content range in the oat-food or drink product or food ingredient is, for example, 10 wt% or less, preferably 5 wt% or less, more preferably 3 wt% or less, still more preferably 2.5 wt% or less, or 2 wt% or less. The protein content range described above may be applied to an oat-food or drink product or food ingredient having any property, and is preferably applied particularly when the oat-food or drink product or food ingredient is a beverage containing oat-derived protein and β-glucan.

The β-glucan content in the oat-food or drink product or food ingredient used in the present invention is not particularly limited, may vary depending on the properties of the food or drink product or food ingredient, and is, for example, 0.01 wt% or more, 0.05 wt% or more, preferably 0.1 wt% or more, more preferably 0.2 wt% or more, still more preferably 0.3 wt% or more, or 0.4 wt% or more. The upper limit of the β-glucan content range in the oat-food or drink product or food ingredient is, for example, 2.5 wt% or less, 2 wt% or less, preferably 1.5 wt% or less, more preferably 1 wt% or less, still more preferably 0.75 wt% or less, or 0.5 wt% or less. The β-glucan content range described above may be applied to an oat-food or drink product or food ingredient having any property, and is preferably applied particularly when the oat-food or drink product or food ingredient is a beverage containing oat-derived protein and β-glucan.

The oat content (oat content is represented as the amount of whole grain oat flour equivalent, and the same applies hereinafter) in an oat-food or drink product or food ingredient used in the present invention is not particularly limited, may vary depending on the properties of the food or drink product or food ingredient, and is, for example, 0.5 wt% or more, 1 wt% or more, 3 wt% or more, preferably 5 wt% or more, 8 wt% or more, more preferably 10 wt% or more, still more preferably 12 wt% or more, or 13 wt% or more. The upper limit of the oat content range in the oat-food or drink product or food ingredient is, for example, 40 wt% or less, 30 wt% or less, preferably 25 wt% or less, more preferably 20 wt% or less, still more preferably 15 wt% or less. The oat content range described above may be applied to an oat-food or drink product or food ingredient having any property, and is preferably applied particularly when the oat-food or drink product or food ingredient is a beverage containing oat-derived protein and β-glucan.

### 1-2. Protein deamidase

The protein deamidase used in the present invention is an enzyme that exhibits an action of degrading an amide group-containing side chain of a protein without cleavage of a peptide bond or crosslinking of the protein, and the kind, origin, and the like thereof are not particularly limited. In addition, as long as the above action is the main activity, it may further have an action of degrading an amide group-containing side chain of a protein accompanied by cleavage of a peptide bond and crosslinking of the protein.

### 1-2-1. Specific examples of protein deamidase

Examples of the protein deamidase include an enzyme that deamidates a glutamine residue in a protein and converts it into glutamic acid (for example, protein glutaminase) and an enzyme that deamidates an asparagine residue in a protein and converts it into aspartic acid (for example, protein asparaginase).

More specific examples of the protein deamidase include protein deamidases derived from the genus Chryseobacterium, the genus Flavobacterium, the genus Empedobacter, the genus Sphingobacterium, the genus Aureobacterium, the genus Myroides, the genus Luteimicrobium, the genus Agromyces, the genus Microbacterium, and the genus Leifsonia or variants thereof. For more specific examples of these protein deamidases, for example, JP 2000-50887 A, JP 2001-218590 A, WO 2006/075772 A1, and WO 2015/133590 can be referred to. These protein deamidases may be used singly or in combination of two or more kinds thereof.

Among these protein deamidases, a protein deamidase derived from the genus Chryseobacterium or a variant thereof is preferred, and a protein glutaminase derived from the genus Chryseobacterium or a variant thereof is more preferred, from the viewpoint of further enhancing the effect of the present invention.

More preferred specific examples of the protein glutaminase derived from the genus Chryseobacterium or a variant thereof include protein glutaminases listed in the following [A] and [B].

[A] Protein glutaminases derived from Chryseobacterium proteolyticum species
[B] Protein glutaminases including a polypeptide described in any of (1) to (3) below:

(1) a polypeptide having an amino acid sequence set forth in SEQ ID NO: 1 or 2,
(2) a polypeptide in which one or several amino acid residues are substituted, added, inserted, or deleted in the amino acid sequence set forth in SEQ ID NO: 1 or 2 and that exhibits heat resistance equivalent to that of a polypeptide having the amino acid sequence set forth in SEQ ID NO: 1 or 2; and
(3) a polypeptide having a sequence identity of 76% or more to the amino acid sequence set forth in SEQ ID NO: 1 or 2 and exhibiting heat resistance equivalent to that of a polypeptide having the amino acid sequence set forth in SEQ ID NO: 1 or 2.

Among the protein glutaminases derived from Chryseobacterium proteolyticum species described in [A], a protein glutaminase derived from Chryseobacterium proteolyticum strain 9670 is preferred from the viewpoint of further improving the effect of the present invention.

The protein glutaminase described in [B] is a protein glutaminase excellent in heat resistance. The polypeptide described in (1) is a protein glutaminase derived from Chryseobacterium sp., and the polypeptides described in (2) and (3) are variants of the polypeptide described in (1). The polypeptides described in (1) to (3) include not only polypeptides obtained by artificial substitution but also polypeptides originally having such an amino acid sequence.

In the polypeptide described in (2), the amino acid modifications to be introduced may include any one of the modifications including substitution, addition, insertion, and deletion (for example, substitution only), or include two or more of the modifications (for example, substitution and insertion). In the polypeptide described in (2), the number of amino acid differences at any difference site may be 1 or several, and is, for example, 1 to 80, preferably 1 to 70, 1 to 60, 1 to 50, 1 to 40, or 1 to 30, more preferably 1 to 20, 1 to 10, 1 to 8, 1 to 7, 1 to 6, 1 to 5, or 1 to 4, still more preferably 1 to 3, particularly preferably 1 or 2, or 1.

In the polypeptide described in (3), the sequence identity to the amino acid sequence set forth in SEQ ID NO:

1 or 2 may be 76% or more, and is preferably 80% or more, more preferably 85% or more, or 88% or more, still more preferably 90% or more, or 93% or more, even still more preferably 95% or more, 96% or more, or 97% or more, particularly preferably 98% or more, or 99% or more.

Here, in the polypeptide described in (3), the sequence identity to each amino acid sequence set forth in SEQ ID NO: 1 or 2 is a sequence identity calculated by comparison with the amino acid sequence set forth in SEQ ID NO: 1 or 2. In addition, the "sequence identity" refers to a value of an amino acid sequence identity obtained by bl2seq program (Tatiana A. Tatsusova, Thomas L. Madden, FEMS Microbiol. Lett., Vol. 174, p 247-250, 1999) in BLAST PACKAGE [sgi 32 bit edition, Version 2.0.12; available from National Center for Biotechnology Information (NCBI)]. Parameter settings may be as follows: Gap insertion Cost value: 11 and Gap extension Cost value: 1.

In the polypeptides described in (2) and (3), the amino acids at position 176 (cysteine), position 217 (histidine), and position 237 (aspartic acid) in the amino acid sequences set forth in SEQ ID NOs: 1 and 2 are considered to be protein glutaminase activity catalytic residues, and therefore it is desirable not to introduce substitutions or deletions to these sites.

In the polypeptides described in (2) and (3), when an amino acid substitution has been introduced to SEQ ID NO: 1 or 2, there may be mentioned conservative substitution as a preferred embodiment of the amino acid substitution to be introduced. That is, examples of the substitution in the polypeptides described in (2) and (3) include the following substitutions: in a case where the amino acid to be substituted is a non-polar amino acid, a substitution with another non-polar amino acid; in a case where the amino acid to be substituted is a non-charged amino acid, a substitution with another non-charged amino acid; in a case where the amino acid to be substituted is an acidic amino acid, a substitution with another acidic amino acid; and in a case where the amino acid to be substituted is a basic amino acid, a substitution with another basic amino acid.

In the polypeptides described in (2) and (3), the phrase "exhibiting heat resistance equivalent to that of a polypeptide having an amino acid sequence set forth in SEQ ID NO: 1 or 2" means that the protein glutaminase residual activity is equivalent to that of the polypeptide described in (1) when the protein glutaminase residual activity at 65°C is measured, specifically, that the protein glutaminase residual activities of the polypeptides described in (2) and (3) are about 0.8 to 1.2 in a case where the protein glutaminase residual activity of the polypeptide described in (1) is defined as 1. The phrase "protein glutaminase residual activity at 65°C" refers to the relative amount (%) of the protein glutaminase activity of an enzyme exposed to the temperature condition of 65°C determined when enzymes are each exposed to the temperature condition of 4°C or 65°C for 10 minutes and the protein glutaminase activity of the enzyme exposed to the temperature condition of 4°C is defined as 100%.

1-2-2. Preparation method of protein deamidase

The protein deamidase can be prepared from a culture solution of a microorganism from which the protein deamidase is derived, or a culture solution of a transformant using a DNA encoding the protein deamidase.

Examples of the transformant using a DNA encoding the protein deamidase include transformants using the DNAs encoding the protein glutaminase described in [B]. As an example of the DNA encoding the protein glutaminase described in [B], there may be mentioned a DNA described in any one of the following (i) to (iii).

(i) A DNA having the base sequence set forth in SEQ ID NO: 3 or 4;

(ii) a DNA having a base sequence that has a homology of 76% or more with the base sequence set forth in SEQ ID NO: 3 or 4 and encodes a polypeptide having protein glutaminase activity; and

(iii) a DNA that hybridizes with a DNA having a base sequence complementary to a DNA having the base sequence set forth in SEQ ID NO: 3 or 4 under stringent conditions and has a base sequence encoding a polypeptide which has protein glutaminase activity

As for the DNA described in (i), the base sequence set forth in SEQ ID NO: 3 is a gene encoding a polypeptide having the amino acid sequence set forth in SEQ ID NO: 1 among the polypeptides described in (1), and the base sequence set forth in SEQ ID NO: 4 is a gene encoding a polypeptide having the amino acid sequence set forth in SEQ ID NO: 2 among the polypeptides described in (1).

As a preferred example of the DNA described in (ii), there may be mentioned a DNA having a base sequence having a homology of 80% or more, more preferably 85% or more, or 88% or more, still more preferably 90% or more, or 93% or more, even still more preferably 95% or more, 96% or more, or 97% or more, particularly preferably 98% or more, or 99% or more with the base sequence set forth in SEQ ID NO: 3 or 4.

Here, the "homology" of DNAs is calculated using publicly available or commercially available software with an algorithm to compare a reference sequence as a query sequence. Specifically, BLAST, FASTA, GENETYX (GENETYX CORPORATION), or the like can be used, and these may be used with a default parameter setting.

In addition, a base sequence in which a mutation by substitution, addition, insertion, or deletion corresponding to the substitution, addition, insertion, or deletion of the amino acid sequence described above has occurred in several bases in the base sequence set forth in SEQ ID NO: 3 or 4 is also included in the DNA described in (ii) as long as it encodes a polypeptide having protein glutaminase activity.

For the DNA described in (iii), the stringent conditions are conditions in which a nylon membrane on which the DNA is immobilized is incubated with a probe in a solution containing 6 × SSC (1 × SSC is obtained by dissolving 8.76 g of sodium chloride and 4.41 g of sodium citrate in 1 liter of water), 1 w/v% SDS, 100 μg/mL salmon sperm DNA, 0.1 w/v% bovine serum albumin, 0.1 w/v% polyvinylpyrrolidone, and 0.1 w/v% Ficoll at 65°C for 20 hours to perform hybridization.

For a detailed procedure of the hybridization method, Molecular Cloning, A Laboratory Manual 2nd ed. (Cold Spring Harbor Laboratory Press (1989)) and the like can be referred to.

Hereinafter, an example of a method for obtaining the DNA of [B] by hybridization will be described, but the method for obtaining the DNA is not limited to the following.

First, a DNA obtained from an appropriate gene source is connected to a plasmid or a phage vector according to a standard method to prepare a DNA library. A transformant obtained by introducing this library into an appropriate host is cultured on a plate, the grown colonies or plaques are transferred to a nitrocellulose or nylon membrane, and the DNA is fixed to the membrane after denaturation treatment. This membrane is incubated in a solution having the above composition containing a probe labeled with $^{32}$P or the like in advance under the above stringent conditions to perform hybridization. As the probe, a polynucleotide encoding all or a part of the amino acid sequence set forth in SEQ ID NO: 1 or 2 can be used.

After completion of hybridization, the non-specifically adsorbed probe is washed away, and a clone hybridized with the probe is identified by autoradiography or the like. This operation is repeated until the hybridized clone is isolated. Finally, a gene encoding a protein having a target enzymatic activity is selected from the obtained clones. The gene can be isolated by a known polynucleotide extraction method such as an alkaline method.

The DNA according to the present invention can also be isolated from a microorganism producing the predetermined protein glutaminase. For example, a target DNA can be isolated from the genome of the microorganism by PCR or a hybridization method performed using a genomic DNA derived from Chryseobacterium sp. as a template and using a primer or a probe designed in consideration of degeneracy of a gene from known amino acid sequence information or a primer or a probe designed based on known base sequence information.

The DNAs according to the present invention encompass various kinds of DNAs due to codon degeneracy. Artificially producing a variety of DNAs encoding the same amino acid sequence can be easily performed using known genetic engineering techniques. For example, in the production of protein by genetic engineering techniques, when a codon used on an original gene encoding a target protein has a low usage frequency in the host, the expression level of the protein may be low. In such a case, high expression of the target protein can be achieved by optimizing the codon usage frequency for the host without changing the encoded amino acid sequence.

As an index representing the codon usage frequency, the total of the host optimal codon usage frequency of each codon may be adopted. The optimal codon is defined as a codon having the highest usage frequency among codons corresponding to the same amino acid. The codon usage frequency is not particularly limited as long as it is optimized for the host, and examples of the optimal codon of Escherichia coli include the following. F: phenylalanine (ttt), L: leucine (ctg), I: isoleucine (att), M: methionine (atg), V: valine (gtg), Y: tyrosine (tat), stop codon (taa), H: histidine (cat), Q: glutamine (cag), N: asparagine (aat), K: lysine (aaa), D: aspartic acid (gat), E: glutamic acid (gaa), S: serine (agc), P: proline (ccg), T: threonine (acc), A: alanine (gcg), C: cysteine (tgc), W: tryptophan (tgg), R: arginine (cgc), G: glycine (ggc).

As a method for artificially modifying an amino acid sequence by introducing a mutation into a gene, a known method such as a Kunkel method or a Gapped duplex method, or a mutation introduction kit using a site-directed mutagenesis method, for example, QuikChange (trademark) Site-Directed Mutagenesis Kit (Agilent Technologies, Inc.), GeneArt (trademark) Site-Directed Mutagenesis System (Thermo Fisher Scientific Inc.), or TaKaRa Site-Directed Mutagenesis System (Mutan-K, Mutan-Super Express Km or the like: Takara Bio Inc.) can be used.

The base sequence of a DNA can be confirmed by sequencing according to a conventional method. For example, sequencing can be carried out by a dideoxynucleotide chain termination method (Sanger et al. (1977) Proc. Natl. Acad. Sci. USA 74: 5463) or the like. In addition, the sequence can be analyzed using an appropriate DNA sequencer.

Whether or not the obtained DNA is a DNA encoding a target protein glutaminase can be confirmed by comparing the determined base sequence with the base sequence set forth in SEQ ID NO: 3 or 4. Alternatively, it can be confirmed by comparing the amino acid sequence estimated from the determined base sequence with the amino acid sequence set forth in SEQ ID NO: 1 or 2.

The DNA of [B] can be linked to a promoter and a terminator to construct an expression cassette, and further, the expression cassette can be inserted into an expression vector to construct a recombinant vector. Alternatively, the DNA of [B] can be inserted into an expression vector to construct a recombinant vector.

The expression cassette or recombinant vector may further include, as necessary, transcriptional elements such as an enhancer, a CCAAT box, a TATA box, and an SPI site, in addition to a promoter and a terminator, as regulatory factors. These regulatory factors may be operably linked to the DNA of [B]. The phrase "being operatively linked" means that various regulatory factors that regulate the DNA of [B] are linked to the DNA of [B] in an operative state in a host cell.

The expression vector is preferably constructed for gene recombination from a phage, plasmid, or virus capable of autonomously proliferating in the host. Such expression vectors are known, and examples of commercially available expression vectors include pQE vectors (QIAGEN), pDR540, pRIT2T (Cytiva), and pET vectors (Merck KGaA). The expression vector may be used in appropriate combination with a selected host cell. Examples of the combination in the case of using Escherichia coli as a host cell include a combination of a pET vector and Escherichia coli strain DH5α, a combination of a pET vector and Escherichia coli strain BL21(DE3), and a combination of a pDR540 vector and Escherichia coli strain JM109.

A transformant is obtained by introducing the expression cassette or recombinant vector into a host and transforming the host.

The host used for the production of the transformant is not particularly limited as long as the gene can be introduced, the expression cassette or the recombinant vector is stable, the host can autonomously proliferate, and the characters of the gene containing the incorporated DNA can be expressed. Suitable examples of the host include bacteria belonging to the genus Escherichia such as Escherichia coli, the genus Bacillus such as Bacillus subtilis, and the genus Pseudomonas such as Pseudomonas putida; and a yeast, and the host may also be an animal cell, insect cell, plant cell, or the like.

Specific examples of the method for introducing an expression cassette or a recombinant vector include a recombinant vector method and a genome editing method. Conditions for introducing the expression cassette or the recombinant vector into the host may be appropriately set according to the kind of the host and the like. In the case of using a bacterium as a host, examples of the method include a method using competent cells by calcium ion treatment and an electroporation method. In the case of using a yeast as a host, examples of the method include an electroporation method, a spheroplast method, and a lithium acetate method. In the case of using an animal cell as a host, examples of the method include an electroporation method, a calcium phosphate method, and a lipofection method. In the case of using an insect cell as a host, examples of the method include a calcium phosphate method, a lipofection method, and an electroporation method. In the case of using a plant cell as a host, examples of the method include an electroporation method, an Agrobacterium method, a particle gun method, and a PEG method.

Whether or not the expression cassette or the recombinant vector according to the present invention is incorporated into the host can be confirmed by a PCR method, a Southern hybridization method, a Northern hybridization method, or the like. When it is confirmed whether or not the expression cassette or the recombinant vector according to the present invention has been incorporated into the host by the PCR method, for example, the genomic DNA, the expression cassette, or the recombinant vector may be separated and purified from the transformant.

For example, when the host is a bacterium, the separation and purification of the expression cassette or the recombinant vector are performed based on a lysate obtained by lysing the bacterium. As a method of lysis, for example, treatment is performed with a lytic enzyme such as lysozyme, and if necessary, a protease, other enzymes, and a surfactant such as sodium lauryl sulfate (SDS) are used in combination.

Furthermore, freeze-thaw and a physical crushing method such as French press treatment may be combined. The separation and purification of the DNA from the lysate can be performed, for example, by deproteinization through phenol treatment and protease treatment, ribonuclease treatment, alcohol precipitation treatment, and a commercially available kit being appropriately combined.

DNA cleavage can be performed according to a conventional method, for example, using restriction enzyme treatment. As the restriction enzyme, for example, a type II restriction enzyme that acts on a specific nucleotide sequence is used. The DNA and the expression cassette or the expression vector are bound using, for example, a DNA ligase.

Thereafter, a primer specific to the introduced DNA is designed using the separated and purified DNA as a template, and PCR is performed. The amplification product obtained by PCR is subjected to agarose gel electrophoresis, polyacrylamide gel electrophoresis, capillary electrophoresis, or the like, and stained with ethidium bromide, SYBR Green solution, or the like, and then the amplification product is detected as a band, whereby transformation can be confirmed.

The amplification product can be detected by performing PCR using a primer labeled with a fluorescent dye or the like in advance. Furthermore, a method of binding an amplification product to a solid phase such as a microplate and confirming the amplification product by fluorescence, an enzymatic reaction, and the like may also be adopted.

The method for collecting the protein deamidase from a culture solution of a microorganism from which the protein deamidase is derived, or a culture solution of a transformant using a DNA encoding the protein deamidase is not particularly limited. For example, in the case of using a microorganism that secretes a protein deamidase, an enzyme can be separated and/or purified after recovering bacterial cells from the culture solution in advance by filtration, a centrifugal treatment, or the like, as necessary. In the case of using a microorganism that does not secret a protein deamidase, an enzyme can be separated and/or purified after recovering bacterial cells from the culture solution in advance as necessary and then disrupting the bacterial cells by pressurization treatment, an ultrasonic treatment, or the like to expose the enzyme. As an enzyme separation and/or purification method, a known protein separation and/or purification method can be used without particular limitation, and examples thereof include a centrifugal separation method, a UF concentration method, a salting-out method, and various chromatography methods using an ion exchange resin and the like. The separated and/or purified enzyme can be pulverized by a drying method such as freeze-drying or reduced-pressure drying, and can also be pulverized using an appropriate excipient and/or drying aid in the drying method. The separated and/or purified enzyme can also be liquefied by adding an appropriate additive and subjecting it to filtration sterilization.

1-2-3. Amount of protein deamidase used

The amount of the protein deamidase used is not particularly limited, but the amount of the protein deamidase used per g of the oat protein is, for example, 0.01 U or more, 0.05 U or more, or 0.1 U or more. From the viewpoint of further enhancing the effect of the present invention, the amount of the protein deamidase used per g of the oat protein is preferably 0.2 U or more, or 0.5 U or more, more preferably 0.75 U or more, or 1 U or more, still more preferably 1.3 U or more, or 1.5 U or more. The upper limit of the range of the amount of the protein deamidase used per g of the oat protein is not particularly limited, and examples thereof include 40 U or less, 30 U or less, 20 U or less, 10 U or less, 5 U or less, 3 U or less, and 2 U or less.

The amount of the protein deamidase used per g of the oat (whole grain oat equivalent, the same applies hereinafter) is, for example, 0.005 U or more, 0.01 U or more, or 0.02 U or more. From the viewpoint of further enhancing the effect of the present invention, the amount of the protein deamidase used per g of the oat is preferably 0.05 U or more, 0.075 U or more, or 0.1 U or more, more preferably 0.125 U or more, or 0.15 U or more, still more preferably 0.175 U or more, or 0.2 U or more. The upper limit of the range of the amount of the protein deamidase used per g of the oat is not particularly limited, and examples thereof include 4 U or less, 3 U or less, 2 U or less, 1 U or less, 0.6 U or less, 0.4 U or less, 0.3 U or less, and 0.25 U or less.

For the activity of the protein deamidase, the amount of enzyme liberating 1 μmol of ammonia per minute using benzyloxycarbonyl-L-glutaminylglycine (Z-Gln-Gly) as a substrate is defined as 1 unit (1 U).

1-3. Enzyme used in combination

In the step of treating, with a protein deamidase, an oat-food or drink product or food ingredient that has not been subjected to heat treatment (that is, pre-heat treatment), another enzyme can be used in combination with the protein deamidase. Examples of the enzyme that can be used in combination therewith include α-amylase and β-amylase. The use of α-amylase and β-amylase in combination therewith is preferred from the viewpoint of improving the solubility of the processed oat-food or drink product or food ingredient, particularly the processed oat beverage which is produced by the present invention.

The α-amylase is not particularly limited, and examples thereof include α-amylases derived from the genus Aspergillus and the genus Bacillus. α-Amylases derived from Bacillus subtilis, Bacillus amyloliquefaciens, and Bacillus licheniformis in the genus Bacillus are preferred, and α-amylase derived from Bacillus amyloliquefaciens is more preferred.

When α-amylase is used in the present invention, it is allowable to use an α-amylase enzyme preparation having contaminating β-glucanase activity. In the present invention, since the protein deamidase is allowed to act under a predetermined temperature condition, even when an α-amylase enzyme preparation having contaminating β-glucanase activity is used in combination with the protein deamidase, the contaminant β-glucanase can be deactivated without deactivating α-amylase. Thus, even when an α-amylase enzyme preparation having contaminating β-glucanase activity is used, the degradation of β-glucan by contaminant β-glucanase can be suppressed.

The β-amylase is not particularly limited, and examples thereof include β-amylases derived from plants (wheat, soy) and the genus Bacillus. β-Amylase derived from the genus Bacillus is preferred, and β-amylase derived from Bacillus flexus species is more preferred.

The amount of α-amylases to be used per part by weight of oat (provided that it is whole grain oat equivalent) is not particularly limited, and for example, the total amount thereof is 0.1 to 20 U or 0.5 to 10 U, preferably 1 to 6 U, 2 to 5 U or 3 to 4 U, more preferably 3.1 to 3.7 U or 3.2 to 3.6 U. For the activity of α-amylase, the amount of enzyme that reduces the color of potato starch caused by iodine by 10% per minute is defined as 1 unit (1 U).

The amount of β-amylases to be used per part by weight of oat (provided that it is whole grain oat equivalent) is not particularly limited, and for example, the total amount thereof is 0.005 to 5 U or 0.01 to 3 U, preferably 0.025 to 1.5 U or 0.05 to 0.75 U, more preferably 0.075 to 0.5 U or 0.1 to 0.3 U. For the activity of β-amylase, the amount of enzyme that provides an increase of the reducing power corresponding to 1 mg of glucose per minute using potato starch as a substrate is defined as 1 unit (1 U).

1-4. Conditions such as reaction conditions

In the present invention, in the step of treating, with a protein deamidase, an oat-food or drink product or food ingredient that has not been subjected to heat treatment (that is, pre-heat treatment), the treatment is performed under a temperature condition of 65 to 75°C. By allowing the protein deamidase to act under the temperature condition of 65 to 75°C, β-glucanase is effectively deactivated, while the protein deamidase itself effectively exerts a protein deamidation action.

Preferred examples of the upper limit of the range of the temperature condition may depend on the heat resistance of the protein deamidase to be used, but it is 73°C or lower, more preferably 70°C or lower from the viewpoint that the effect of the present invention is further enhanced. Another example of the lower limit of the range of the temperature condition is, for example, higher than 65°C, specifically 66°C or higher, 67°C or higher, 68°C or higher, or 69°C or higher, and the effect of the present invention can be favorably obtained even at the above temperature.

When the above enzyme is used in combination with the protein deamidase in the step of treating with the protein deamidase, the enzyme used in combination is allowed to coexist with the protein deamidase in the enzymatic reaction system using the protein deamidase.

The time for treating, with a protein deamidase, an oat-food or drink product or food ingredient that has not been subjected to heat treatment (that is, pre-heat treatment) is not particularly limited, and may be appropriately determined according to a preparation scale or the like, and it is, for example, 30 minutes or longer, preferably 1 hour or longer, more preferably 1.5 hours or longer, and still more preferably 2 hours or longer. The upper limit of the range of the time is not particularly limited, and examples thereof include 12 hours or less, 6 hours or less, 3 hours or less, and 2.5 hours or less.

After completion of the treatment, the enzyme deactivation treatment is performed, followed by cooling and, as necessary post-treatment to obtain a processed oat-food or drink product or food ingredient.

1-5. Processed oat-food or drink product or food ingredient

The processed oat-food or drink product or food ingredient obtained by the production method of the present invention has been processed to be rich in β-glucan because of suppression of β-glucan degradation and to degrade an amide group-containing side chain of a protein. As a specific mode of the form in which the amide group-containing side chain of the protein is degraded, there is a mode, for example, in which the amount of soluble protein is increased (as compared to that contained in the oat as the raw material). In the present invention, a preferred processed oat-food or drink product or food ingredient is a beverage (processed oat beverage), and particularly preferred is processed oat milk.

Furthermore, the obtained processed oat-food or drink product or food ingredient may be prepared as a dried product of the processed oat-food or drink product or food ingredient through a drying step. The drying method is not particularly limited, and examples thereof include freeze drying, vacuum drying, and spray drying. Examples of the shape of the dried product include powder, fine particles, and granules. Such a dried product may be dissolved and/or dispersed in water to prepare an oat beverage, or may be used as a material of various foods other than beverages.

2. Protein deamidation agent used for predetermined applications

As described above, when an oat-food or drink product or food ingredient that has not been subjected to heat treatment (that is, pre-heat treatment) is treated with the protein deamidase under a temperature condition of 65 to 75°C, the β-glucanase is deactivated and the protein deamidase can be allowed to act effectively. Thus, the present invention also provides a protein deamidation agent that contains a protein deamidase and is used under a temperature condition of 65 to 75°C in order to perform protein deamidation of an oat-food or drink product or food ingredient that has not been subjected to heat treatment while suppressing degradation of β-glucan.

In the protein deamidation agent used for the predetermined application, the kind, amount, treatment conditions, and the like of components to be used are as described in the section "1. Method for producing processed oat-food or drink product or food ingredient".

EXAMPLES

Hereinafter, the present invention will be specifically described with reference to the examples, but the present invention is not to be construed as being limited to the following examples.

[Oat-food or drink product or food ingredient used]

Oat flour (protein content 13 wt%, β-glucan content 0.41 wt%) which was a pulverized product of oat (whole grains) was used. This oat flour does not have a thermal history due to heat treatment (pre-heat treatment) in the process of being prepared from the oat.

[Enzymes used]

- Protein glutaminase derived from Chryseobacterium proteolyticum strain 9670 (manufactured by Amano Enzyme Inc.)
- Protein glutaminase derived from strain 61798 of Chryseobacterium sp.
- $\alpha$-Amylase derived from Bacillus amyloliquefaciens (manufactured by Amano Enzyme Inc.)
- $\beta$-Amylase derived from Bacillus flexus (manufactured by Amano Enzyme Inc.)

Hereinafter, a protein glutaminase may also be referred to as "PG".

(Protein glutaminase derived from strain 61798)

(1) Selection of heat-resistant protein glutaminase producing strain

By using protein glutaminase activity and temperature stability as indexes, strain 57594 and strain 61798 of Chryseobacterium sp. were selected from publicly unknown strain libraries owned by the applicant, and stocked in a culture solution containing glycerol.

(2) Culturing of selected strain

The components in Table 1 were dissolved in water so as to have the indicated concentrations, and autoclaved at 121°C for 30 minutes to prepare a medium. The selected strain was inoculated into a culture medium and cultured at 120 rpm at 30°C for 40 to 48 hours. After the culturing, the culture solution was centrifuged to collect bacterial cells, and only the supernatant was collected. Diatomaceous earth was added to the supernatant, which was filtrated, and the culture filtrate was concentrated using an ultrafiltration membrane.

(3) Purification of enzymes

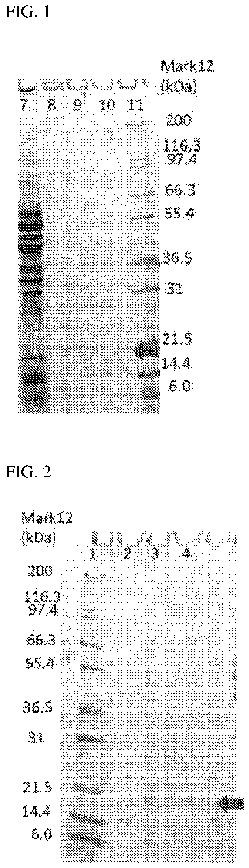

The concentrated solution was subjected to salting out, treated with Phenyl-sepharose, and Sephacryl S-100 to purify the enzyme. SDS-PAGE images of the concentrated fractions after purification of the culture solutions of strain 57594 and strain 61798 are shown in Fig. 1 and Fig. 2, respectively. In Fig. 1, Lane 7 represents a pre-purification fraction, Lanes 8 to 10 represent purified fractions from the strain 57594 culture, and Lane 11 represents a molecular weight marker. In Fig. 2, Lane 1 represents a molecular weight marker, and Lanes 2 to 4 represent purified fractions from the strain 61798 culture. As shown in Fig. 1 and Fig. 2, 18 kDa (band indicated by an arrow) was confirmed as a purified product in the purified fraction.

[Table 1]

| Lactose - 3 media | % (w/v) |
|---|---|
| Lactose monohydrate (FUJIFILM Wako Pure Chemical) | 0.5 |
| Bacto peptone (Difco) | 2 |
| $Na_2HPO_4 \cdot 12H_2O$ | 0.34 |
| $KH_2PO_4$ | 0.025 |
| $MgSO_4 \cdot 7H_2O$ | 0.025 |
| $FeSO_4 \cdot 7H_2O$ | 0.005 |
| pH 7.2 | |

(4) Enzymological property evaluation test

The 18 kDa purified fractions obtained from the culture solution of strain 57594 and the culture solution of strain 61798 (protein glutaminase derived from strain 57594 and protein glutaminase derived from strain 61798) were evaluated for the following enzymological properties. Similarly, the existing protein glutaminase derived from Chryseobacterium proteolyticum strain 9670 was evaluated for the following enzymological properties.

(4-1) Measurement of protein glutaminase activity

- Benzyloxycarbonyl-L-glutaminylglycine (Z-Gln-Gly; PEPTIDE INSTITUTE, Inc.) was dissolved in 0.2 mol/L phosphate buffer (pH 6.5) to prepare a 30 mmol/L solution, and the solution was used as a substrate solution.
- In a test tube, 0.1 mL of an enzyme solution whose activity was to be measured was placed, and allowed to stand in a constant temperature water bath at 37 $\pm$ 0.5°C for 1 minute. Then, 1 mL of a substrate solution previously allowed to stand at 37 $\pm$ 0.5°C for 10 minutes was added, and the mixture was immediately mixed.
- This solution was allowed to stand for 10 minutes to perform an enzymatic reaction, and then 1 mL of a 0.4 mol/L trichloroacetic acid solution was added to stop the enzymatic reaction.
- A measurement blank was prepared by adding 0.1 mL of an enzyme solution to a test tube, and adding 1 mL of a 0.4 mol/L trichloroacetic acid solution and 1 mL of a substrate solution in this order.
- A color reaction by Ammonia Test Wako (FUJIFILM Wako Pure Chemical Corporation) was performed, and ammonia released by an enzymatic reaction for 10 minutes was quantified on the basis of a value of absorbance at a wavelength of 630 nm.
- The amount of enzyme that produces 1 $\mu$mol ammonia per minute was defined as 1 unit (1 U), and the activity value was calculated from the amount of ammonia released by the enzymatic reaction.

(4-2) Temperature stability test

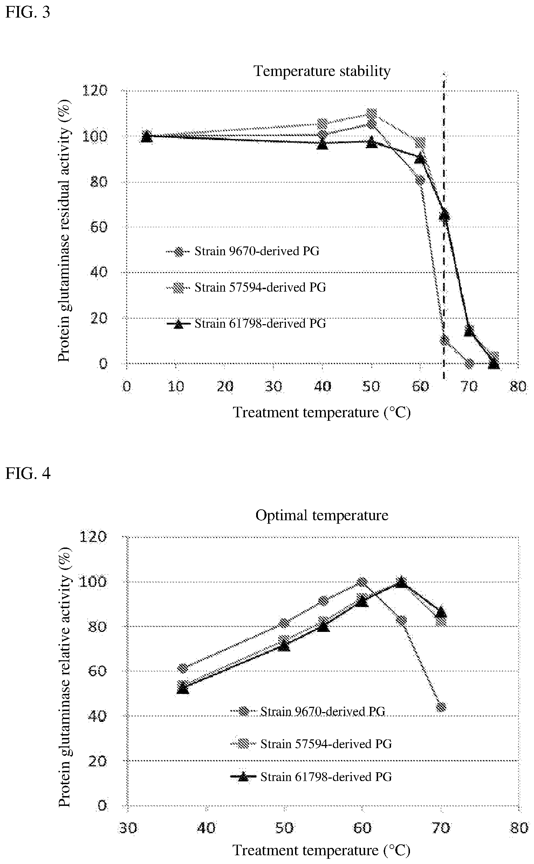

The purified fraction was substituted with a 0.2 mol/L phosphate buffer (pH 6.5), and allowed to stand at 4°C for 10 minutes or heated at 40°C, 50°C, 60°C, 65°C, 70°C, or 75°C for 10 minutes. Thereafter, according to the method described in (4-1), an enzymatic reaction was performed to measure the protein glutaminase activity. In a case where the protein glutaminase activity of the purified fraction exposed to the condition of 4°C was defined as 100%, the relative amount of the protein glutaminase activity of the purified fraction exposed to the heating condition at each temperature was calculated as the protein glutaminase residual activity (%). The results are shown in Fig. 3. As shown in Fig. 3, while the residual activity of the PG derived from Chryseobacterium proteolyticum strain 9670 at 65°C was 11%, the residual activity of the protein glutaminase derived from strain 57594 was 65%, and the residual activity of the protein glutaminase derived from strain 61798 was 66%.

(4-3) Optimum temperature test

The protein glutaminase activity was measured in the same manner as in (4-1), except that the enzymatic reaction temperature was changed to 37°C, 50°C, 55°C, 60°C, 65°C, or 70°C. In a case where the protein glutaminase activity under the temperature condition at which the maximum activity of each purified fraction was exhibited (60°C for the PG derived from Chryseobacterium proteolyticum strain 9670, 65°C for the PG derived from strain 57594 and the PG derived from strain 61798) was defined as 100%, the relative amount of the protein glutaminase activity under each temperature condition was calculated as the protein glutaminase relative activity (%). The results are shown in Fig. 4. As shown in Fig. 4, while the optimal temperature of the PG derived from Chryseobacterium proteolyticum strain 9670 was 60°C, the optimal temperatures of the protein glutaminases derived from strain 57594 and strain 61798 were both 65°C.

(4-4) pH Stability test

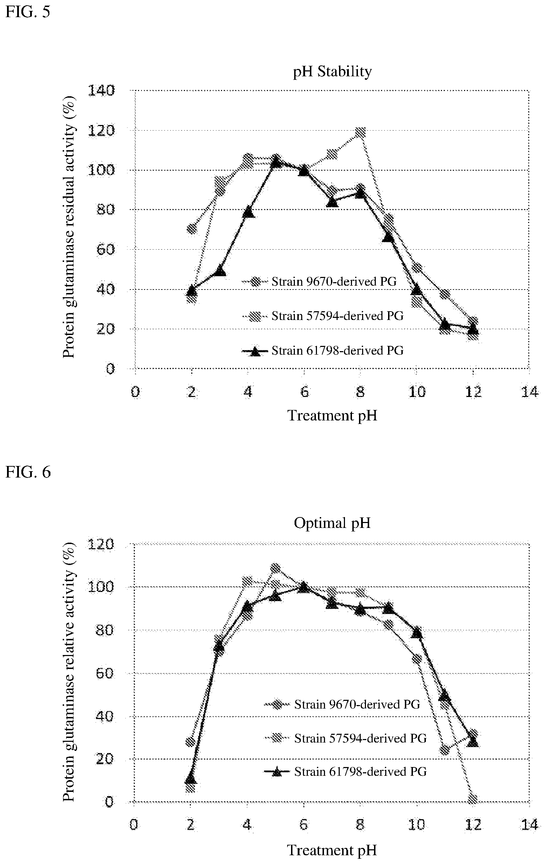

The purified fraction was adjusted to pH 2, 3, 4, 5, 6, 7, 8, 9, 10, 11, or 12 with a Britton-Robinson buffer, and allowed to stand at 37°C overnight (18 hours) under each pH. Thereafter, according to the method described in (4-1), an enzymatic reaction was performed to measure the protein glutaminase activity. In a case where the protein glutaminase activity in the case of pH 6 was defined as 100%, the relative amount of the protein glutaminase activity in the purified fraction exposed to each pH condition was calculated as the protein glutaminase residual activity (%). The results are shown in Fig. 5.

(4-5) Optimal pH test

The protein glutaminase activity was measured in the same manner as in (4-1) except that the pH of the substrate solution was adjusted to pH 2, 3, 4, 5, 6, 7, 8, 9, 10, 11, or 12 with a Britton-Robinson buffer, and the enzymatic reaction was performed under each pH. In a case where the protein glutaminase activity in the case of pH 6 was defined as 100%, the relative amount of the protein glutaminase activity under each pH condition was calculated as the protein glutaminase relative activity (%). The results are shown in Fig. 6.

(5) Sequence

Degenerate primers were designed from sequence information of Chryseobacterium bacteria estimated to be closely related to strain 57594 and strain 61798. Gene fragments were amplified with PrimeSTAR (registered trademark) GXL DNA Polymerase (Takara Bio Inc.) using genomic DNAs of strain 57594 and strain 61798 as templates and the degenerate primers. The amplified fragments were collected by NucleoSpin (registered trademark) Gel and PCR Clean-up (Takara Bio Inc.) and sequence analysis using the degenerate primers was performed. As a result, the amino acid sequence of the protein glutaminase derived from strain 57594 (SEQ ID NO: 1); the base sequence around the protein glutaminase gene derived from strain 57594 including structural gene (SEQ ID NO: 3) encoding SEQ ID NO: 1 (SEQ ID NO: 5); the amino acid sequence of the protein glutaminase derived from strain 61798 (SEQ ID NO: 2); and the base sequence around the protein glutaminase gene derived from strain 61798 including structural gene (SEQ ID NO: 4) encoding SEQ ID NO: 2 (SEQ ID NO: 6) were determined. Among the obtained protein glutaminases, strain 61798 was subjected to the following tests.

[Method for measuring enzymatic activity]

(1) Method for measuring protein deamidase activity

The activity of the protein glutaminase was measured as in "(4-1) Measurement of protein glutaminase activity" of "(4) Enzymological property evaluation test" in "[Enzymes used]".

(2) Method for measuring α-amylase activity

A 1% potato starch substrate solution (0.1 mol/L acetic acid (pH 5.0)), 10 mL, was heated at 37°C for 10 minutes, then 1 mL of a sample solution containing α-amylase was added, and the mixture was immediately shaken. This solution was allowed to stand at 37°C for 10 minutes, then 1 mL of this solution was added to 10 mL of a 0.1 mol/L hydrochloric acid test solution, and the mixture was immediately shaken. Next, 0.5 mL of this solution was measured out, and 10 mL of a 0.0002 mol/L iodine test solution (JP) was added thereto. The mixture was shaken, and then absorbance (AT) at a wavelength of 660 nm was measured using water as a control. Separately, 1 mL of water was added instead of the sample solution, and the same procedure was carried out to measure the absorbance (AB). The amount of enzyme that reduced the color of potato starch caused by iodine by 10% per minute was defined as 1 unit (1 U).

$$[\text{Mathematical Formula 1}]$$

$$\alpha\text{-Amylase activity (U/g, U/mL)} = (AB - AT)/AB \times 1/W$$

AT: absorbance of reaction solution
AB: absorbance of blank solution
W: sample amount (g or mL) in 1 mL of sample solution

(3) Method for measuring β-amylase activity

A potato starch was used as a substrate, and the potato starch was previously dried at 105°C for 2 hours. Then, 1.0 g of the dried product was weighed out, 20 mL of water was added, and 5 mL of a sodium hydroxide test solution (2 mol/L) was gradually added thereto while stirring to form a paste. Next, the mixture was heated in a water bath for 3 minutes with stirring, and then 25 mL of water was added thereto. After cooling, the mixture was neutralized by adding a hydrochloric acid test solution (2 mol/L) and a hydrochloric acid test solution (0.1 mol/L), 10 mL of a 1 mol/L acetic acid-sodium acetate buffer solution (pH 5.0) was added, and water was further added to make 100 mL, thereby obtaining a substrate solution.

The substrate solution, 10 mL, was measured out, and warmed at 37°C for 10 minutes, 1 mL of the sample solution was added thereto. The mixture was immediately shaken and warmed at the same temperature for 10 minutes, after which 4 mL of a Fehling's solution was further added thereto. The mixture was shaken lightly, heated in a water bath for 15 minutes, then cooled to 25°C or lower, and 2 mL of a potassium iodide solution (for β-amylase invertase activity test) and 2 mL of sulfuric acid (1→6) were added thereto. There was obtained a test solution. Separately, the same procedure as in the preparation of the test solution was carried out using 10 mL of water instead of the substrate solution to prepare a comparative solution. For the test solution and the comparative solution, liberated iodine was titrated with a 0.05 mol/L sodium thiosulfate solution. The endpoint in titration was defined as the point at which the blue color generated by adding 1 to 2 drops of a soluble starch test solution near the endpoint disappeared.

The amount of enzyme that provides an increase of the reducing power corresponding to 1 mg of glucose per minute was defined as 1 unit (1 U), and calculated from the following formula.

[Mathematical Formula 2]

$$\beta\text{-Amylase activity (U/g, U/mL)} = \text{glucose amount (mg)} \times 1/10 \times 1/M$$
$$\text{Glucose amount (mg)} = (b - a) \times 1.6 \times f$$

a : titration value (mL) of enzymatic reaction solution
b : titration value (mL) of blank solution
1.6 : 1 mL of 0.05 mol/L sodium thiosulfate solution is corresponding to 1.6 mg of glucose amount
1/10 : unit conversion coefficient for reaction time (min)
M : sample amount (g or mL) in 1 mL of sample solution
f : factor for 0.05 mol/L sodium thiosulfate solution (for quantification)

[Test example]

(1) Method

Oat flour, 1.35 g, was suspended in 10 mL of tap water to prepare an aqueous oat flour suspension (oat milk). The details of the composition of the prepared aqueous oat flour suspension are shown in Table 2. The aqueous oat flour suspension was heated to 60°C, 65°C, or 70°C with stirring, 0.7 mg (4.55 U) of α-amylase, 0.4 mg (0.26 U) of β-amylase, and the indicated amount of the PG in Table 3 were added, and an enzymatic reaction was performed for 120 minutes. After the reaction, the reaction mixture was boiled for 10 minutes to deactivate the PG, and cooled at room temperature. In this manner, processed oat milk was obtained.

[Table 2]

| | |
|---|---|
| Oat (based on whole grain) concentration | 13.5 wt% |
| Oat-derived protein content | 1.76 wt% |
| Oat-derived β-glucan content | 0.41 wt% |

[Table 3]

| | Strain 9670-derived PG | Strain 61798-derived PG |
|---|---|---|
| Weight concentration in aqueous oat flour suspension | 1.02 ppm | 1.22 ppm |
| Activity value per g of oat-derived protein | 1.50 U | 1.50 U |
| Activity value per g of oat (based on whole grain) | 0.20 U | 0.20 U |

(2) Measurement of β-glucan content

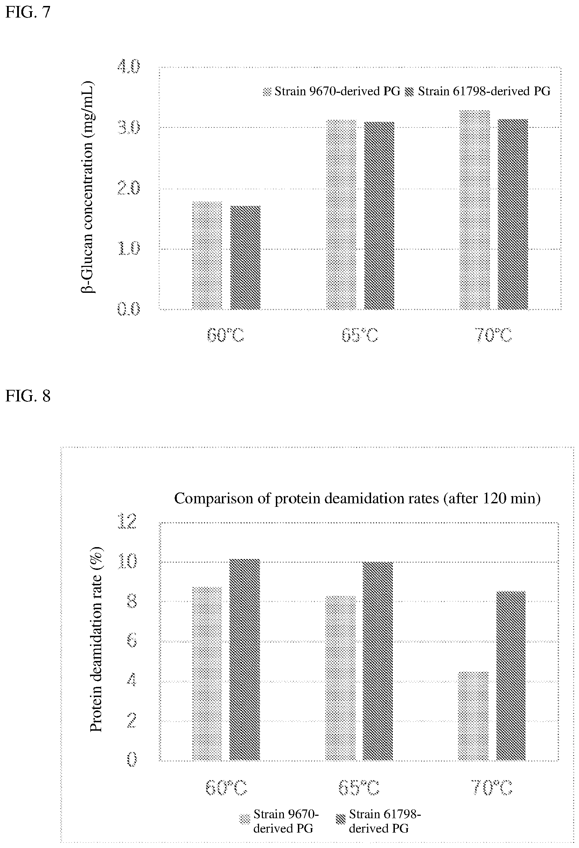

For the obtained processed oat milk, the β-glucan content was quantified using β-Glucan Assay Kit (Mixed Linkage) (Megazyme Ltd.). The results are shown in Fig. 7.

(3) Measurement of protein deamidation rate

For the obtained processed oat milk, the amount of free ammonia was quantified using Ammonia Test Wako (FUJIFILM Wako Pure Chemical Corporation). As for the aqueous oat flour suspension (oat milk) used as the material, 3N concentrated sulfuric acid was added to and mixed with the oat flour water suspension, and the mixture was treated at 110°C for 3 hours, then cooled, neutralized with 6N sodium hydroxide, and centrifuged to obtain a supernatant. For the obtained supernatant, the amount of free ammonia was quantified using Ammonia Test Wako (FUJIFILM Wako Pure Chemical Corporation). The amount of free ammonia in the processed oat milk was divided by the amount of free ammonia in the aqueous oat flour suspension (oat milk) as a raw material to derive the protein deamidation rate.

(4) Results

As is apparent from Fig. 7, it was confirmed that the amount of β-glucan was significantly large when the reaction temperature was 65°C and 70°C as compared to when the reaction temperature was 60°C. That is, in the reaction system of this test example, it was suggested that β-glucanase was deactivated at 65°C or higher.

On the other hand, as is apparent from Fig. 8, it was confirmed that the deamidation reaction effectively occurred, that is, the protein glutaminase was able to effectively act without being deactivated even at reaction temperatures of 65°C and 70°C which were sufficiently high temperatures to deactivate β-glucanase. When the reaction temperature was 70°C, the deamidation rate by the high heat-resistant PG derived from strain 61798 was remarkably high.

**Claims**

1. A method for producing a processed oat-food or drink product or food ingredient, the method comprising the step for treating an oat-food or drink product or food ingredient that has not been subjected to heat treatment, with a protein deamidase under a temperature condition of 65 to 75°C.

2. The method according to claim 1, wherein the protein deamidase is a protein glutaminase derived from Chryseobacterium proteolyticum.

3. The method according to claim 1, wherein the protein deamidase is a protein glutaminase comprising a polypeptide described in any of (1) to (3) below:

    (1) a polypeptide having an amino acid sequence set forth in SEQ ID NO: 1 or 2;
    (2) a polypeptide in which one or several amino acid residues are substituted, added, inserted, or deleted in the amino acid sequence set forth in SEQ ID NO: 1 or 2 and that exhibits heat resistance equivalent to heat resistance of a polypeptide having the amino acid sequence set forth in SEQ ID NO: 1 or 2; and
    (3) a polypeptide having a sequence identity of 76% or more to the amino acid sequence set forth in SEQ ID NO: 1 or 2 and exhibiting heat resistance equivalent to heat resistance of a polypeptide having the amino acid sequence set forth in SEQ ID NO: 1 or 2.

4. The method according to claim 1, wherein the oat-food or drink product or food ingredient is oat milk.

5. A protein deamidation agent that comprises a protein deamidase and is used under a temperature condition of 65 to 75°C in order to perform protein deamidation of an oat-food or drink product or food ingredient that has not been subjected to heat treatment while suppressing degradation of β-glucan.

FIG. 1

FIG. 2

FIG. 3

Temperature stability

FIG. 4

Optimal temperature

FIG. 5

pH Stability

FIG. 6

Optimal pH

FIG. 7

FIG. 8

Comparison of protein deamidation rates (after 120 min)

## INTERNATIONAL SEARCH REPORT

| International application No. |
|---|
| **PCT/JP2022/020989** |

### A. CLASSIFICATION OF SUBJECT MATTER

*A23L 7/104*(2016.01)i; *C12N 9/78*(2006.01)i; *C12N 15/31*(2006.01)i; *C12N 15/55*(2006.01)i
FI: A23L7/104; C12N15/55; C12N15/31; C12N9/78 ZNA

According to International Patent Classification (IPC) or to both national classification and IPC

### B. FIELDS SEARCHED

Minimum documentation searched (classification system followed by classification symbols)

A23L7/104; C12N9/78; C12N15/31; C12N15/55

Documentation searched other than minimum documentation to the extent that such documents are included in the fields searched

Published examined utility model applications of Japan 1922-1996
Published unexamined utility model applications of Japan 1971-2022
Registered utility model specifications of Japan 1996-2022
Published registered utility model applications of Japan 1994-2022

Electronic data base consulted during the international search (name of data base and, where practicable, search terms used)

JSTPlus/JMEDPlus/JST7580 (JDreamIII); CAplus/MEDLINE/FSTA/AGRICOLA/BIOSIS/BIOTECHNO/CABA/S CISEARCH/TOXCENTER (STN)

### C. DOCUMENTS CONSIDERED TO BE RELEVANT

| Category* | Citation of document, with indication, where appropriate, of the relevant passages | Relevant to claim No. |
|---|---|---|
| X | JP 2016-506732 A (OATLY AB) 07 March 2016 (2016-03-07) paragraphs [0016]-[0019], [0024], [0026]-[0027], [0029], examples 2, 4 | 1, 4-5 |
| Y | paragraphs [0016]-[0019], [0024], [0026]-[0027], [0029], examples 2, 4 | 2-3 |
| Y | WO 2021/049591 A1 (AMANO ENZYME INC) 18 March 2021 (2021-03-18) paragraphs [0008], [0014]-[0015] | 2-3 |
| A | YAMAGUCHI, S. et al. Protein-glutaminase from Chryseobacterium proteolyticum, an enzyme that deamidates glutaminyl residues in proteins Purification, characterization and gene cloning. Eur. J. Biochem. 268. 1410-1421(2001) abstract, fig. 5 | 1-5 |
| P, X | CN 112956540 A (BEIJING OATOAT FOOD TECHNOLOGY CO., LTD.) 15 June 2021 (2021-06-15) fig. 1 | 1, 4-5 |
| P, Y | fig. 1 | 2-3 |
| P, A | WO 2022/045152 A1 (AMANO ENZYME INC) 03 March 2022 (2022-03-03) examples | 1-5 |

☑ Further documents are listed in the continuation of Box C.   ☑ See patent family annex.

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| **25 July 2022** | **09 August 2022** |

| Name and mailing address of the ISA/JP | Authorized officer |
|---|---|
| **Japan Patent Office (ISA/JP)** **3-4-3 Kasumigaseki, Chiyoda-ku, Tokyo 100-8915 Japan** | |
| | Telephone No. |

Form PCT/ISA/210 (second sheet) (January 2015)

**INTERNATIONAL SEARCH REPORT**

International application No.

**PCT/JP2022/020989**

**C.     DOCUMENTS CONSIDERED TO BE RELEVANT**

| Category* | Citation of document, with indication, where appropriate, of the relevant passages | Relevant to claim No. |
|---|---|---|
| P, A | WO 2021/251344 A1 (AMANO ENZYME INC) 16 December 2021 (2021-12-16) examples | 1-5 |

Form PCT/ISA/210 (second sheet) (January 2015)

**INTERNATIONAL SEARCH REPORT**

| International application No. |
|---|
| **PCT/JP2022/020989** |

---

**Box No. I      Nucleotide and/or amino acid sequence(s) (Continuation of item 1.c of the first sheet)**

---

1. With regard to any nucleotide and/or amino acid sequence disclosed in the international application, the international search was carried out on the basis of a sequence listing:

   a. ☑ forming part of the international application as filed:

      ☑ in the form of an Annex C/ST.25 text file.

      ☐ on paper or in the form of an image file.

   b. ☐ furnished together with the international application under PCT Rule 13*ter*.1(a) for the purposes of international search only in the form of an Annex C/ST.25 text file.

   c. ☐ furnished subsequent to the international filing date for the purposes of international search only:

      ☐ in the form of an Annex C/ST.25 text file (Rule 13*ter*.1(a)).

      ☐ on paper or in the form of an image file (Rule 13*ter*.1(b) and Administrative Instructions, Section 713).

2. ☐ In addition, in the case that more than one version or copy of a sequence listing has been filed or furnished, the required statements that the information in the subsequent or additional copies is identical to that forming part of the application as filed or does not go beyond the application as filed, as appropriate, were furnished.

3. Additional comments:

Form PCT/ISA/210 (continuation of first sheet) (January 2015)

**INTERNATIONAL SEARCH REPORT**
Information on patent family members

International application No.

**PCT/JP2022/020989**

| Patent document cited in search report | | | Publication date (day/month/year) | Patent family member(s) | | | Publication date (day/month/year) |
|---|---|---|---|---|---|---|---|
| JP | 2016-506732 | A | 07 March 2016 | US | 2015/0351432 | A1 | |
| | | | | paragraphs [0013]-[0016], [0020], [0022]-[0023], [0025]-[0026], examples 2, 4 | | | |
| | | | | WO | 2014/123466 | A1 | |
| | | | | EP | 2953482 | A1 | |
| | | | | AU | 2014215729 | A | |
| | | | | CA | 2899717 | A | |
| | | | | KR | 10-2015-0113200 | A | |
| | | | | CN | 105007757 | A | |
| | | | | HK | 1212562 | A | |
| | | | | RU | 2015125371 | A | |
| | | | | SG | 11201504862Q | A | |
| | | | | DK | 2953482 | T | |
| | | | | HR | P20190518 | T | |
| | | | | RS | 58501 | B | |
| | | | | NZ | 709990 | A | |
| | | | | ES | 2719698 | T | |
| | | | | PL | 2953482 | T | |
| | | | | PT | 2953482 | T | |
| WO | 2021/049591 | A1 | 18 March 2021 | (Family: none) | | | |
| CN | 112956540 | A | 15 June 2021 | (Family: none) | | | |
| WO | 2022/045152 | A1 | 03 March 2022 | (Family: none) | | | |
| WO | 2021/251344 | A1 | 16 December 2021 | (Family: none) | | | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2002527089 A
- US 7494683 B
- JP 2000050887 A
- JP 2001218590 A
- WO 2006075772 A1
- WO 2015133590 A

**Non-patent literature cited in the description**

- **TATIANA A. TATSUSOVA ; THOMAS L. MADDEN.** *FEMS Microbiol. Lett.,* 1999, vol. 174, 247-250
- Molecular Cloning, A Laboratory Manual. Cold Spring Harbor Laboratory Press, 1989
- **SANGER et al.** *Proc. Natl. Acad. Sci. USA,* 1977, vol. 74, 5463